# EUROPEAN PATENT APPLICATION

(11) **EP 2 437 033 A2**
(43) Date of publication of application: **04.04.2012**
(21) Application number: 11182833.1
(22) Date of filing: 26.09.2011
(51) Int. Cl.: G01C 23/00

(54) **DYNAMIC TASK AND ADAPTIVE AVIONICS DISPLAY MANAGER**

(30) Priority: 29.09.2010 US 387710 P; 09.08.2011 US 206409
(71) Applicant: Honeywell International, Inc., Morristown, New Jersey 07962-2245 (US)
(72) Inventor: Keinrath, Claudia, Morristown, NJ 07962-2245 (US); Dorneich, Michael Christian, Morristown, NJ 07962-2245 (US); Whitlow, Stephen, Morristown, NJ 07962-2245 (US); Passinger, Bretislav, Morristown, NJ 07962-2245 (US); Vasek, Jiri, Morristown, NJ 07962-2245 (US); Krupansky, Petr, Morristown, NJ 07962-2245 (US)
(74) Representative: Buckley, Guy Julian

(57) **Abstract**

A system and method are provided for intelligently managing the avionics display, information, and controls to more evenly distribute pilot task loads and/or automatically configure/reconfigure displays during flights.

## Description

### PRIORITY CLAIMS

This application claims the benefit of U.S. Provisional Application No. 61/387,710 filed September 29, 2010.

### TECHNICAL FIELD

The present invention generally relates to adaptive systems, and more particularly relates to systems and methods for adapting avionics display, information, controls and tasks to balance workload.

### BACKGROUND

Operators of complex systems, such as aircraft, are often faced with a challenging work environment where their task load varies between very low where they can become inattentive and drowsy and very high where they can become overloaded and prone to poor performance. Vacillating between under-load and overload can increase stress in operators which can have potentially adverse consequences. Further, operators in these environments are often faced with frequent distractions. In many operational environments with multiple operators, there may be periods where there is inequitable task loading between the operators.

The design goals for next generation air traffic management (ATM) are to increase system capacity by allowing pilots and airlines more responsibility to manage routes, aircraft separation, and to generally have more authority to make changes to the flight profile. However, pilots and airlines may also be responsible for flying more precise routes, planning further ahead, and coordinating with other aircraft to resolve potential conflicts. These changes may result in the need for more information and more automation on the flight-deck to handle the increased complexity, increased precision, and increased flight deck responsibilities. Changes may occur more rapidly, more information may be available, and pilot workload may potentially increase. Currently, pilots can spend a considerable amount of time configuring and reconfiguring their displays as the flight progresses, in order to access the information needed to perform the tasks within the current phase of the flight.

Hence, there is a need for intelligent management of the avionics display, information, and controls to more evenly distribute pilot task load and/or automatically configure/reconfigure displays during flights. The present invention addresses at least these needs.

### BRIEF SUMMARY

In one embodiment, a method for selectively and adaptively reconfiguring one or more of a plurality of flight deck displays in an aircraft includes processing sensor data representative of a current workload state of a pilot, and processing aircraft avionics data representative of a current state of the aircraft. Based on at least one of the processed sensor data and the processed aircraft avionics data, a determination is made as to whether one or more events have occurred. The reconfiguration of one or more of the plurality of flight deck displays is selectively commanded based on the determination of whether the one or more events have occurred.

In another embodiment, a system for selectively and adaptively reconfiguring one or more of a plurality of flight deck displays in an aircraft includes a plurality of workload sensors, an aircraft avionics data source, and a processor. Each of the workload sensors is configured to sense a parameter representative of pilot workload level and supply sensor data representative thereof. The aircraft avionics data source is configured to supply data representative of a current state of the aircraft. The processor is in operable communication with the flight deck displays and is coupled to receive the sensor data and the aircraft avionics data. The processor is configured, upon receipt of at least one of the sensor data and the aircraft avionics data, to determine whether one or more events have occurred, and based on the determination, selectively command reconfiguration of one or more of the plurality of flight deck displays.

In still another embodiment, a method for dynamically managing aircraft flight crew tasks includes processing sensor data representative of a current workload state of a pilot, processing aircraft avionics data representative of a current state of the aircraft, and processing aircraft mission data representative of a current mission state of the aircraft. A determination is made, based on at least one of the processed sensor data, the processed aircraft avionics data, historical task schedules, and the processed aircraft mission data, of a current and future task load of the pilot; and based on the current and future task load of the pilot, a recommendation that the pilot complete one or more tasks is selectively generated.

In yet another embodiment, a system for dynamically managing aircraft flight crew tasks includes a plurality of workload sensors, an aircraft avionics data source, an aircraft mission data source, and a processor. Each of the workload sensors is configured to sense a parameter representative of pilot workload level and supply sensor data representative thereof. The aircraft avionics data source is configured to supply aircraft state data representative of a current state of the aircraft. The aircraft mission data source is configured to supply aircraft mission data representative of a current mission state of the aircraft. The processor is coupled to receive at least one of the sensor data, the aircraft state data, and the aircraft mission data, and is configured, upon receipt thereof, to determine a current and future task load of the pilot and, based on the current and future task load of the pilot, selectively generate a recommendation that the pilot complete one or more tasks.

Furthermore, other desirable features and characteristics of the methods and systems will become apparent from the subsequent detailed description of the invention, taken in conjunction with the accompanying drawings and this background.

### BRIEF DESCRIPTION OF THE DRAWINGS

The present invention will hereinafter be described in conjunction with the following drawing figures, wherein like numerals denote like elements, and wherein:

FIG. 1 depicts a functional block diagram of an example embodiment of a dynamic task and adaptive display management system; and

FIG. 2 depicts a process, in flowchart form, that may be implemented in the flight crew workload management system of FIG. 1.

### DETAILED DESCRIPTION

The following detailed description is merely exemplary in nature and is not intended to limit the invention or the application and uses of the invention. As used herein, the word "exemplary" means "serving as an example, instance, or illustration." Thus, any embodiment described herein as "exemplary" is not necessarily to be construed as preferred or advantageous over other embodiments. All of the embodiments described herein are exemplary embodiments provided to enable persons skilled in the art to make or use the invention and not to limit the scope of the invention which is defined by the claims. Furthermore, there is no intention to be bound by any expressed or implied theory presented in the preceding technical field, background, brief summary, or the following detailed description.

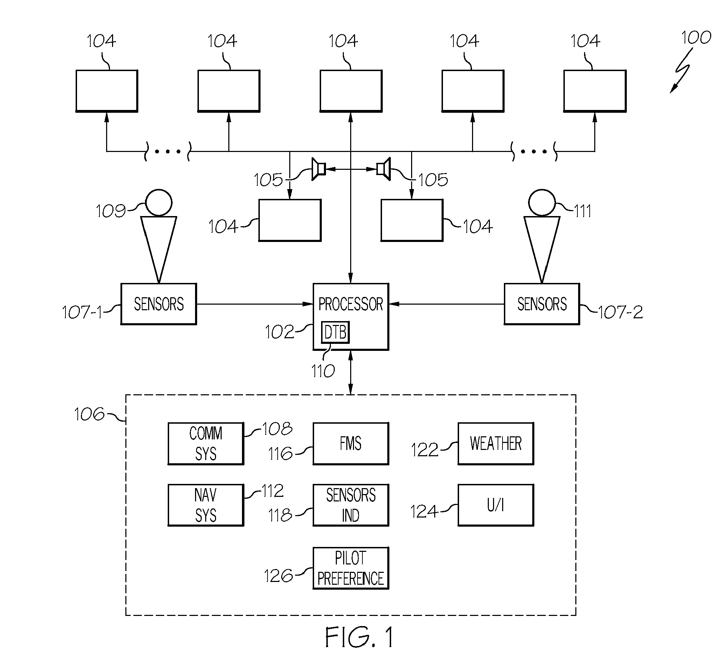

Referring to FIG. 1, a functional block diagram of an example embodiment of a dynamic task and adaptive display management system 100 is depicted, and includes a processor 102, a plurality of displays 104, and a plurality of data sources 106. The processor 102 is in operable communication with the display devices 104 and the data sources 106. The processor 102 is coupled to receive various types of data from the data sources 106, and may be implemented using any one (or a plurality) of numerous known general-purpose microprocessors or application specific processor(s) that operates in response to program instructions. The processor 102 may be implemented using various other circuits, not just a programmable processor. For example, digital logic circuits and analog signal processing circuits could also be used. In this respect, the processor 102 may include or cooperate with any number of software programs (e.g., avionics display programs) or instructions designed to carry out various methods, process tasks, calculations, and control/display functions described below.

The display devices 104 are used to display various images and data, in a graphic, iconic, and a textual format, and to supply visual feedback to the pilot 109 and the co-pilot 111. It will be appreciated that the display device 104 may be implemented using any one of numerous known displays suitable for rendering graphic, iconic, and/or text data in a format viewable by the pilot 109 and co-pilot 111. Non-limiting examples of such displays include various cathode ray tube (CRT) displays, and various flat panel displays, such as various types of LCD (liquid crystal display), TFT (thin film transistor) displays, and OLED (organic light emitting diode) displays. The display may additionally be based on a panel mounted display, a HUD projection, or any known technology. In an exemplary embodiment, display device 104 includes a panel display. It is further noted that the system 100 could be implemented with more than one display device 104. For example, the system 100 could be implemented with two or more display devices 104.

No matter the number or particular type of display that is used to implement the display devices 104, it was noted above that the processor 102 is responsive to the various data it receives to render various images on the display devices 104. The images that the processor 102 renders on the display devices 104 will depend, for example, on the type of display being implemented. For example, the display devices 104 may implement one or more of a multi-function display (MFD), a three-dimensional MFD, a primary flight display (PFD), a synthetic vision system (SVS) display, a vertical situation display (VSD), a horizontal situation indicator (HSI), a traffic awareness and avoidance system (TAAS) display, a three-dimensional TAAS display, just to name a few. Moreover, and as FIG. 1 depicts in phantom, the system 100 may be implemented with multiple display devices 104, each of which may implement one or more of these different, non-limiting displays. The display device 106 may also be implemented in an electronic flight bag (EFB) and, in some instance, some or all of the system 100 may be implemented in an EFB.

The data sources 106 may vary in type and number, but in the depicted embodiment include various avionics systems. Some non-limiting examples of avionics systems that may comprise the data sources 106 include communication systems 108, navigation and guidance systems 112, flight management systems 116, sensors and indicators 118, weather systems 122, and various user interfaces 124 to assist the pilot 109 and co-pilot 111 in implementing control, monitoring, communication, and navigation functions of the aircraft.

As FIG. 1 further depicts, the data sources 106 may also include, at least in some embodiments, a pilot preference data source 126. The pilot preference data source 126, if included, includes data representative of individual pilot 109 and co-pilot 111 preferences, behaviors, habits, and tendencies associated with avionics settings and configurations. The settings and configurations may include, but are not limited to, display management, automation preferences, and avionics settings. These preferences may also include temporal and contextual elements regarding when to make changes to the settings and configurations. The pilot preference data may also include training and operational history. Such data may include, but are not limited to, hours flown, aircraft flown, recentness of experience, airports, approaches, runways, and facilities used, and training completed and recentness of the training.

The system 100 may additionally include a plurality of audio output devices 105. The audio output devices 105, if included, may be variously implemented. No matter the specific implementation, each audio output device 105 is preferably in operable communication with the processor 102. The processor 102, or other non-depicted circuits or devices, supplies analog audio signals to the output devices 105. The audio devices 105, in response to the analog audio signals, generate audible sounds. The audible sounds may include speech (actual or synthetic) or generic sounds or tones associated with alerts and notifications.

The processor 102, as noted above, is in operable communication with the data sources 106 and thus receives data representative of the state of the pilot 109 and co-pilot 111, the state of the aircraft, and the state of the aircraft mission. The processor 102 is configured, in response to these data, to selectively and adaptively command a reconfiguration of one or more of the displays 104 during the flight. More specifically, and in a particular embodiment, the processor 102, based on the received data, determines whether an event (or combination of events and/or factors) has occurred that should trigger a display reconfiguration. The event (or combination of events and/or factors) may include, but is not limited to, phase of flight, pilot workload, a step in a procedure, pilot experience with the destination airport, or an alert (audio, visual, or both), just to name a few.

The configuration changes to one or more displays 104 may be implemented automatically or in response to an input from the pilot 109 and/or co-pilot. This may depend, for example, on factors such as the relative criticality of current tasks, time sensitivity of tasks, pilot workload, and so on. The changes to the displays 104 may be governed by information needed to support the current task(s), and could be within a single display or across multiple displays (including visual, auditory, and haptic). Moreover, when the system 100 is configured to include the pilot preference data source 126, the setup of the displays 104 may be modified accordingly.

It will be appreciated that many different types of information could be used to trigger a display re-configuration. For example, certain phases of flight have distinct events that initiate new pilot actions and that may call for different display setups. As a specific example, at top-of-decent (TOD) pilots may change the navigation display to be north-up, and to enable weather, traffic, and terrain layers. Yet another example, which is more time critical, is configuring displays to support a missed approach when time is short and pilots may be reconfiguring displays under stress.

The system 100 is additionally configured such that display changes are preferably adaptive in that the level of automation may vary depending, for example, on pilot workload. As such, and as FIG. 1 further depicts, the system 100 may additionally include a plurality of sensors 107 (e.g., pilot sensors 107-1, co-pilot sensors 107-2). The sensors 107, which may be variously implemented, are configured to sense and supply physiological data, contextual data, and/or various other relevant data to the processor 102. The sensors 107 may be located on the body and/or clothing of the pilot 109 and co-pilot 111, and/or on one or more other devices (e.g., helmet, eye wear) worn by the pilot 109 and co-pilot 111. Alternatively, the sensors 107 may be disposed nearby the pilot 109 and co-pilot 111.

It will be appreciated that the number and type of sensors 107 may vary. Some non-limiting examples of suitable physiological sensors 107 include an electroencephalogram (EEG) sensor, an electrocardiogram (ECG) sensor, an electro-oculogram (EOG) sensor, an impedance pneumogram (ZPG) sensor, a galvanic skin response (GSR) sensor, a blood volume pulse (BVP) sensor, a respiration sensor, an electromyogram (EMG) sensor, a pupilometry sensor, a visual scanning sensor, a blood oxygenation sensor, a blood pressure sensor, a skin and core body temperature sensor, a near-infrared optical brain imaging sensor, or any other device that can sense physiological changes in the pilot.

The EEG sensors monitor the pilot's and co-pilot's brain wave activity by sensing electrical potential at the scalp. Measurements by the EEG sensors are categorized into frequency bands, including delta, theta, alpha, and beta. For example, the delta band ranging from 1-4Hz indicates a state of unconsciousness, the theta band ranging from 4-8Hz indicates a state of daydreaming, the alpha band ranging from 8-13Hz indicates an alert, but not mentally busy state, and the beta band ranging from 13-30 Hz indicates a state of higher thought process. Other frequency bands are possible. Based on the location of the EEG sensors, and the dominant frequencies detected, EEG data may help evaluate the type and amount of mental activity of the pilot 109 and co-pilot 111. For example, if there are significant brain waves measured in the frontal brain, the pilot 109 or co-pilot 111 may be actively manipulating information within their working memory. As a result, the EEG sensors may be used to measure the cognitive state of the pilot 109 and co-pilot 111.

Other physiological sensors mentioned above include ECG sensors, EOG sensors, ZPG sensors, GSR sensors, pupilometry sensors, visual scanning sensors, blood oxygenation sensors, BVP sensors, EMG sensors, blood pressure sensors, and near-infrared optical brain imaging sensors. The ECG sensors measure heart rate by detecting electrical activity of the heart muscle. The EOG sensors measure eye movement by detecting electrical changes between the front and back of the eye as the eye moves. The ZPG sensors (or other type of respiration sensors) measure lung capacity and can be used to determine whether the pilot 109 or co-pilot 111 is having difficulty breathing. The GSR sensors measure changes in conductivity of the skin caused by sweating and saturation of skin ducts prior to sweating. The pupilometry sensors measure pupil dilation to determine the level of engagement or interest in a task, or cognitive load of a task. The visual scanning sensors measure scanning behavior and dwell time to provide insight into visual attention. The blood oxygenation sensors sense oxygen levels in the blood. The BVP sensors measure heart rate by detecting changes in blood volume at a given location of the body. The EMG sensors measure currents associated with muscle action. The near-infrared optical brain imaging sensors measure brain function.

The sensors 107 may additionally include an accelerometer, an eye tracker, or any other device that can sense contextual data. The devices may be commercial off-the-shelf devices or custom designed. The accelerometers, if included, measure the rate at which an object is moving, the acoustic sensors, if included, measure the loudness and frequency of ambient sounds, and the eye trackers, if included, measure pupilometry and/or visual scanning behavior. Data from the accelerometers may be used to measure head movement such as yaw, pitch, and roll. Data from the eye trackers may be used to infer cognitive state from pupil dilation response and to infer visual attention indices from dwell time and scanning patterns.

No matter the specific number and type of sensors 107 used, each sensor 107 supplies data representative of the measured stimuli to the processor 102. It will be appreciated that the data may be transmitted to the processor 102 wirelessly or via hardwired connections, and that the data may be modified, prior to transmission, to format the data as needed. The processor 102, upon receipt of the sensor data, assesses the individual workload and/or fatigue state of both the pilot 109 and the co-pilot 111. It will be appreciated that the pilot and co-pilot workload and/or fatigue states may be assessed using any one of numerous known methods. An example of one particular methodology is disclosed in U.S. Patent No. 7,454,313, entitled "Hierarchical Workload Monitoring for Optimal Subordinate Tasking," which is assigned to the assignee of the instant invention, and which is hereby incorporated by reference in its entirety.

Before proceeding further, it is noted that workload may also be assessed from secondary (i.e. non-direct) sources, such as tracking response times to stimuli (e.g. alerts) or performance on tasks.

For example, during relatively high workload periods, the displays 104 may be reconfigured automatically, without pilot interaction. However, during relatively low or normal workload periods, any display changes/reconfigurations may require permission from the pilot before the change/reconfiguration occurs. In some embodiments, display changes/reconfigurations may require permission regardless of the relative workload level of the pilot. As noted above, other information, such as pilot preferences may also be used. For example, changes may be initiated if, based on pilot data, the system 100 is aware of the pilot familiarity (or lack of familiarity) with the airport.

In addition to adaptively (and selectively) reconfiguring one or more of the displays 104, the processor 102 may also implement a function that is referred to herein as a dynamic task balancer (DTB) 110. The DTB 110 reasons on *a priori* knowledge of operations and current operational context, using data supplied from at least selected ones of the data sources 106, to intelligently schedule selective tasks to better balance pilot 109 and co-pilot 111 workloads across the mission.

The DTB 110, based on data received from the data sources 106, estimates the task loads of the pilot 109 and co-pilot 111. The estimates may be derived from tracking pilot 109 and co-pilot 111 interaction with system 100, directly sensing the task loads of the pilot 109 and co-pilot 111 (e.g., via sensors 107), or historical estimates. If derived from historical estimates, the system 100 may additionally include a task tracking database 128 that receives and stores data representative of historical pilot/co-pilot interactions with the flight deck. As an illustrative example, historical task characteristics, time estimates, trends and loads, along with current operating context derived from the data sources 106, may provide a reasonable estimate to act as a trigger for the DTB 110. Based on rough timing, system interaction record, and/or spatial location, the DTB 110 can establish a rough estimate of the current mission status on some nominal mission timeline. By reasoning on current and future task load, the DTB 110 can recommend that the pilot 109 and co-pilot 111 undertake one or more tasks early or at a different time in order to balance workload.

The particular tasks that the DTB 110 may recommend may vary, and may depend on various factors. For example, if task load is currently low, there is an upcoming high workload period, and there are tasks that can be done early, then the DTB 110 may recommend that the pilot 109 and/or co-pilot 111 complete one of those tasks. The target task could be selected by estimate to completion time, task priority, or how well it meshes with ongoing tasks. If the task load is currently high, the high task load period is continuing, current tasks are amenable to automated execution, and there are outstanding tasks that can be automated, then the DTB 110 may recommend automatic intervention to reduce current task load. If task load is currently high, and there is an outstanding high priority task with an approaching deadline, then the DTB 110 may generate a reminder to the pilot 109 and/or co-pilot 111 of the high priority task.

In some embodiments, the DTB 110 may support current task tracking. This allows the DTB 110 to understand what tasks the pilot 109 and/or co-pilot 111 are doing currently. To balance task loads, the DTB 110 may be configured to reason on current and projected task responsibilities as well as pilot 109 and co-pilot 111 capabilities to dynamically schedule tasks between the pilot 109 and co-pilot 111. The DTB 110 may also consider current operational context, such as weather, to anticipate a likely increase in task time over the historical average for those tasks impacted by weather. The DTB 100 may also be configured to update task timing and order of execution in an ongoing basis to further refine its responsiveness to changes in operational practices. The DTB 110 could also operate from fixed task time estimates.

Many tasks have soft timing constraints. That is, the task just needs to be completed prior to some deadline. One example of such a task is the final approach from TOD. If the pilot 109 is experiencing low task load prior to a period of anticipated high workload, the DTB 110 may suggest, via a display 104 or an audio device 105, that the pilot 109 complete one of the tasks having a variable timing constraint. This would not only mentally engage the pilot 109, which may be helpful if the pilot 109 is drowsy or bored, but it may also alleviate future high workload and provide a more even balancing of workload across a mission. This proactive workload balancing will minimize periods where the pilot 109 and/or co-pilot 111 may need to react to new and/or greater task demands.

In addition to proactive workload balancing, the DTB 110 may also be configured to support reactive workload balancing by engaging additional automation to reduce current high workload. An additional benefit provided by the DTB, is that it may allow operators, when sufficient time is available, to initiate tasks, carefully go through tasks, and finish the tasks. This can increase the overall quality of operator on-task performance. This DTB 110 may also be configured to generate reminders (audio, visual, or both) of which tasks are of a higher operational priority than others, thus re-directing the pilot 109 and/or co-pilot 111 if they have been distracted by lower priority tasks.

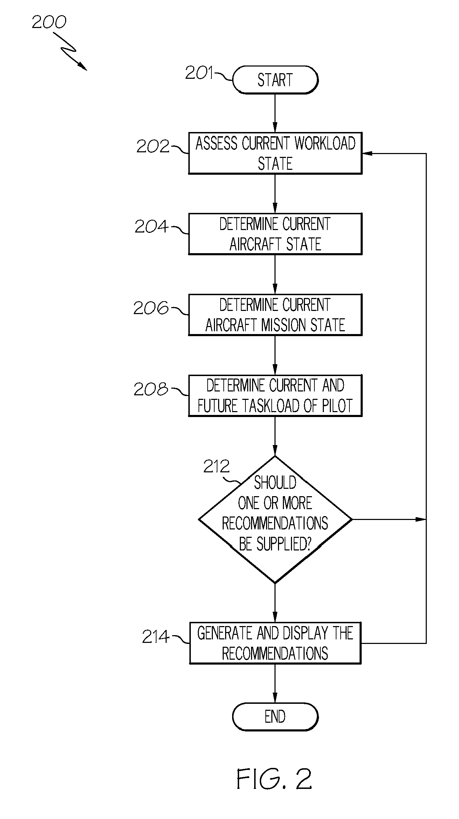

The general methodology implemented in the DTB 110 that was described above is depicted in flowchart form in FIG. 2. For completeness, a description of this method 200 will now be provided. In doing so, it is noted that the parenthetical references refer to like-numbered flowchart blocks.

The method 200 begins by assessing the current workload state of the pilot (202). As noted above, the processor 102 is configured to implement this functionality by processing the sensor data supplied from the sensors 107. The current state of the aircraft (204) and the current mission state of the aircraft (206) are also determined. Thereafter, the current and future task loads of the pilot are determined (208). It is noted that the DTB 110 may be configured to make this determination based on one, two, or all three of these data. No matter which data are used, the DTB 110 will then determine, based on the current and future task load of the pilot, if one or more recommendations should be supplied to the pilot to complete one or more tasks (212). If so, then the recommendations are generated (214).

The various illustrative logical blocks, modules, and circuits described in connection with the embodiments disclosed herein may be implemented or performed with a general purpose processor, a digital signal processor (DSP), an application specific integrated circuit (ASIC), a field programmable gate array (FPGA) or other programmable logic device, discrete gate or transistor logic, discrete hardware components, or any combination thereof designed to perform the functions described herein. A general-purpose processor may be a microprocessor, but in the alternative, the processor may be any conventional processor, controller, microcontroller, or state machine. A processor may also be implemented as a combination of computing devices, e.g., a combination of a DSP and a microprocessor, a plurality of microprocessors, one or more microprocessors in conjunction with a DSP core, or any other such configuration. The word "exemplary" is used exclusively herein to mean "serving as an example, instance, or illustration." Any embodiment described herein as "exemplary" is not necessarily to be construed as preferred or advantageous over other embodiments.

The steps of a method or algorithm described in connection with the embodiments disclosed herein may be embodied directly in hardware, in a software module executed by a processor, or in a combination of the two. A software module may reside in RAM memory, flash memory, ROM memory, EPROM memory, EEPROM memory, registers, hard disk, a removable disk, a CD-ROM, or any other form of storage medium known in the art. An exemplary storage medium is coupled to the processor such the processor can read information from, and write information to, the storage medium. In the alternative, the storage medium may be integral to the processor. The processor and the storage medium may reside in an ASIC. The ASIC may reside in a user terminal. In the alternative, the processor and the storage medium may reside as discrete components in a user terminal.

In this document, relational terms such as first and second, and the like may be used solely to distinguish one entity or action from another entity or action without necessarily requiring or implying any actual such relationship or order between such entities or actions. Numerical ordinals such as "first," "second," "third," etc. simply denote different singles of a plurality and do not imply any order or sequence unless specifically defined by the claim language. The sequence of the text in any of the claims does not imply that process steps must be performed in a temporal or logical order according to such sequence unless it is specifically defined by the language of the claim. The process steps may be interchanged in any order without departing from the scope of the invention as long as such an interchange does not contradict the claim language and is not logically nonsensical.

Furthermore, depending on the context, words such as "connect" or "coupled to" used in describing a relationship between different elements do not imply that a direct physical connection must be made between these elements. For example, two elements may be connected to each other physically, electronically, logically, or in any other manner, through one or more additional elements.

While at least one exemplary embodiment has been presented in the foregoing detailed description of the invention, it should be appreciated that a vast number of variations exist. It should also be appreciated that the exemplary embodiment or exemplary embodiments are only examples, and are not intended to limit the scope, applicability, or configuration of the invention in any way. Rather, the foregoing detailed description will provide those skilled in the art with a convenient road map for implementing an exemplary embodiment of the invention. It being understood that various changes may be made in the function and arrangement of elements described in an exemplary embodiment without departing from the scope of the invention as set forth herein.

## Claims

1. A method for selectively and adaptively reconfiguring one or more of a plurality of flight deck displays in an aircraft, comprising the steps of:
processing sensor data representative of a current workload state of a pilot;
processing aircraft avionics data representative of a current state of the aircraft;
determining, based on at least one of the processed sensor data and the processed aircraft avionics data, whether one or more events have occurred; and
selectively commanding reconfiguration of one or more of the plurality of flight deck displays based on the determination of whether the one or more events have occurred.

2. The method of Claim 1, further comprising:
processing aircraft mission data representative of a current mission state of the aircraft; and
determining whether the one or more events have occurred based additionally on the aircraft mission data.

3. The method of Claim 1, further comprising:
selectively reconfiguring the one or more cockpit displays automatically in response to commanding the reconfiguration thereof.

4. The method of Claim 1, further comprising:
processing pilot preference data, the pilot preference data including information representative of pilot preferences, behaviors, habits, biases, idiosyncrasies, and tendencies associated with at least flight deck display settings and configurations; and
selectively commanding the reconfiguration of one or more of the plurality of flight deck displays based additionally on the pilot preference data.

5. A system for selectively and adaptively reconfiguring one or more of a plurality of flight deck displays in an aircraft, comprising:
a plurality of workload sensors, each of the workload sensors configured to (i) sense a parameter representative of pilot workload level and (ii) supply sensor data representative thereof;
an aircraft avionics data source configured to supply data representative of a current state of the aircraft; and
a processor in operable communication with the flight deck displays and coupled to receive the sensor data and the aircraft avionics data, the processor configured, upon receipt of at least one of the sensor data and the aircraft avionics data, to:
determine whether one or more events have occurred, and
based on the determination, selectively command reconfiguration of one or more of the plurality of flight deck displays.

6. A method for dynamically managing aircraft flight crew tasks, comprising:
processing sensor data representative of a current workload state of a pilot;
processing aircraft avionics data representative of a current state of the aircraft;
processing aircraft mission data representative of a current mission state of the aircraft; and
determining, based on at least one of the processed sensor data, the processed aircraft avionics data, and the processed aircraft mission data, a current and future task load of the pilot; and
based on the current and future task load of the pilot, selectively generating a recommendation that the pilot complete one or more tasks.

7. The method of Claim 6, wherein the sensor data representative of the current workload state of the pilot are derived from tracking pilot interaction with an aircraft flight deck system.

8. The method of Claim 6, wherein the step of selectively generating a recommendation comprises:
determining if the current task load of the pilot is below a first predetermined level;
determining if the future task load of the pilot is going to be above a second predetermined level; and
recommending that the pilot complete one or more target tasks before the future task load is above the second predetermined level.

9. The method of Claim 8, further comprising:
determining an operational priority of current and one or more future tasks; and
selectively generating a reminder of future tasks that are or higher operational priority than current tasks.

10. A system for dynamically managing aircraft flight crew tasks, comprising the steps of:
a plurality of workload sensors, each of the workload sensors configured to (i) sense a parameter representative of pilot workload level and (ii) supply sensor data representative thereof;
an aircraft avionics data source configured to supply aircraft state data representative of a current state of the aircraft;
an aircraft mission data source configured to supply aircraft mission data representative of a current mission state of the aircraft; and
a processor coupled to receive at least one of the sensor data, the aircraft state data, and the aircraft mission data, and configured, upon receipt thereof, to:
determine a current and future task load of the pilot, and
based on the current and future task load of the pilot, selectively generate a recommendation that the pilot complete one or more tasks.
